# EUROPEAN PATENT SPECIFICATION

(11) **EP 4 183 342 B1**
(45) Date of publication and mention of the grant of the patent: **20.08.2025**
(21) Application number: 22208487.3
(22) Date of filing: 21.11.2022
(51) Int. Cl.: A61B 5/287, A61B 5/339, A61B 5/367, A61B 5/00

(54) **MAPPING SYSTEM WITH REALTIME ELECTROGRAM OVERLAY**
ABBILDUNGSSYSTEM MIT ECHTZEIT-ELEKTROGRAMMÜBERLAGERUNG
SYSTÈME DE MAPPAGE AVEC SUPERPOSITION D'ÉLECTROGRAMMES EN TEMPS RÉEL

(30) Priority: 22.11.2021 US 202117532980
(43) Date of publication of application: 24.05.2023
(73) Proprietor: BIOSENSE WEBSTER (ISRAEL) LTD., 2066717 Yokneam (IL)
(72) Inventor: GOVARI, Assaf, Yokneam 2066717 (IL); KATZ, Natan Sharon, Yokneam 2066717 (IL); GLINER, Vadim, Yokneam 2066717 (IL); DVORKIN, Vladimir, Yokneam 2066717 (IL); ALTMANN, Andres Claudio, Yokneam 2066717 (IL); COHEN, Benjamin, Yokneam 2066717 (IL)
(74) Representative: Carpmaels & Ransford LLP

(56) References cited:
- US-A1- 2009 177 111
- US-A1- 2018 242 868
- US-A1- 2019 099 078
- US-A1- 2020 367 829
- US-B1- 6 728 562

## Description

### FIELD OF THE INVENTION

The present invention relates to medical systems, and in particular, but not exclusively, to catheter devices, according to the appended claims.

### BACKGROUND

A wide range of medical procedures involve placing probes, such as catheters, within a patient's body. Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. These methods and systems are described in U.S. Pat. Nos. 5,391,199, 6,690,963, 6,484,118, 6,239,724, 6,618,612 and 6,332,089, in PCT International Publication No. WO 1996/005768, and in U.S. Patent Application Publications Nos. 2002/0065455 and 2003/0120150 and 2004/0068178. Locations may also be tracked using impedance or current based systems.

One medical procedure in which these types of probes or catheters have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Such ablation can cease or modify the propagation of unwanted electrical signals from one portion of the heart to another. The ablation process destroys the unwanted electrical pathways by formation of non-conducting lesions. Various energy delivery modalities have been disclosed for forming lesions, and include use of microwave, laser, pulsed field, and more commonly, radiofrequency energies to create conduction blocks along the cardiac tissue wall. In a two-step procedure, mapping followed by ablation, electrical activity at points within the heart is typically sensed and measured by advancing a catheter containing one or more electrical sensors into the heart, and acquiring data at a multiplicity of points. These data are then utilized to select the endocardial target areas at which the ablation is to be performed.

Electrode catheters have been in common use in medical practice for many years, see for example U.S. Patent Application 2018/242868 A1. They are used to stimulate and map electrical activity in the heart and to ablate sites of aberrant electrical activity. In use, the electrode catheter is inserted into a major vein or artery, e.g., femoral vein, and then guided into the chamber of the heart of concern. A typical ablation procedure involves the insertion of a catheter having a one or more electrodes at its distal end into a heart chamber. A reference electrode may be provided, generally taped to the skin of the patient or by means of a second catheter that is positioned in or near the heart. RF (radio frequency) current is applied between the tip electrode(s) of the ablating catheter, and the reference electrode, flowing through the media between the electrodes it, i.e., blood and tissue. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive.

Therefore, when placing an ablation or other catheter within the body, particularly near the endocardial tissue, it is desirable to have the distal tip of the catheter in direct contact with the tissue. The contact can be verified, for example, by measuring the contact between the distal tip and the body tissue. U.S. Patent Application Publication Nos. 2007/0100332, 2009/0093806 and 2009/0138007 describe methods of sensing contact pressure between the distal tip of a catheter and tissue in a body cavity using a force sensor embedded in the catheter.

A number of references have reported methods to determine electrode-tissue contact, including U.S. Patents 5,935,079; 5,891,095; 5,836,990; 5,836,874; 5,673,704; 5,662,108; 5,469,857; 5,447,529; 5,341,807; 5,078,714; and Canadian Patent Application 2,285,342. A number of these references, e.g., U.S. patents 5,935,079, 5,836,990, and 5,447,529 determine electrode-tissue contact by measuring the impedance between the tip electrode and a return electrode. As disclosed in the '529 patent, it is generally known than impedance through blood is generally lower that impedance through tissue. Accordingly, tissue contact has been detected by comparing the impedance values across a set of electrodes to premeasured impedance values when an electrode is known to be in contact with tissue and when it is known to be in contact only with blood. U.S. Patent Application 2020/367829 A1 describes a medical system, including a catheter configured to be inserted into a chamber of a heart of a living subject, and including catheter electrodes configured to contact tissue at respective locations within the chamber of the heart, and processing circuitry configured to receive signals from the catheter. In an embodiment, the system includes body-surface electrodes configured to be applied to a skin surface of the living subject and the processing circuitry is configured to measure an indication of electrical impedances between the body-surface electrodes and the catheter electrodes, and compute position coordinates of the catheter electrodes responsively to the indication of the electrical impedances.

US Patent 9168004 to Gliner, at al. describes using machine learning to determine catheter electrode contact. The '004 Patent describes cardiac catheterization being carried out by memorizing a designation of a contact state between an electrode of the probe and the heart wall as an in-contact state or an out-of-contact state, and making a series of determinations of an impedance phase angle of an electrical current passing through the electrode and another electrode, identifying maximum and minimum phase angles in the series, and defining a binary classifier adaptively as midway between the extremes. A test value is compared to the classifier as adjusted by a hysteresis factor, and a change in the contact state is reported when the test value exceeds or falls below the adjusted classifier.

US Patent Publication 2013/0085416 of Mest describes a method for the in vivo re-calibration of a force sensing probe such as an electrophysiology catheter which provides for the generation of an auto zero zone. The distal tip of the catheter or other probe is placed in a body cavity within the patient. Verification that there is no tissue contact is made using electrocardiogram (ECG) or impedance data, fluoroscopy or other real-time imaging data and/or an anatomical mapping system. Once verification that there is no tissue contact made, the system recalibrates the signal emanating from the force sensor setting it to correspond to a force reading of zero grams and this recalibrated baseline reading is used to generate and display force readings based on force sensor data.

### SUMMARY

The present invention provides medical systems and methods in accordance with the appended claims. There is provided in accordance with an embodiment of the present disclosure, a medical system, including a catheter configured to be inserted into a chamber of a heart of a living subject, and including a distal end including catheter electrodes configured to contact tissue at respective locations within the chamber of the heart, at least one position sensor configured to provide at least one position signal indicative of a position of the distal end, a display, and processing circuitry configured to compute the position of the distal end of the catheter responsively to the at least one position signal, render to the display a three-dimensional (3D) anatomical map of the chamber of the heart and a 3D representation of the distal end of the catheter, and render to the display over at least part of the anatomical map, at least one intracardiac electrogram (IEGM) trace representing electrical activity in the tissue that is sensed by at least one of the catheter electrodes.

Further in accordance with an embodiment of the present disclosure the processing circuitry is configured to render to the display over at least part of the anatomical map, multiple IEGM traces representing electrical activity in the tissue that is sensed by respective ones of the catheter electrodes.

Still further in accordance with an embodiment of the present disclosure the processing circuitry is configured to receive signals from the catheter, and in response to the signals assess a respective quality of contact of each of at least a sub-set of the catheter electrodes with the tissue, and render to the display multiple IEGM traces representing electrical activity in the tissue that is sensed by respective ones of the catheter electrodes responsively to the respective quality of contact of each of the respective ones of the catheter electrodes with the tissue of the heart at the respective locations.

Additionally in accordance with an embodiment of the present disclosure the processing circuitry is configured to render to the display the multiple IEGM traces representing electrical activity in the tissue that is sensed by respective ones of the catheter electrodes responsively to the respective quality of contact of each of the respective ones of the catheter electrodes with the tissue of the heart at the respective locations exceeding a threshold quality of contact.

Moreover in accordance with the medical system of the present invention the processing circuitry is configured to render to the display the multiple IEGM traces representing electrical activity in the tissue that is sensed by n respective ones of the catheter electrodes responsively to the respective quality of contact of each of the n respective ones of the catheter electrodes with the tissue of the heart being among n highest qualities of contact of the at least sub-set of the catheter electrodes.

Further in accordance with an embodiment of the present disclosure the processing circuitry is configured to receive a user input selecting the sub-set of the catheter electrodes.

Still further in accordance with an embodiment of the present disclosure the processing circuitry is configured to receive a user input selecting a region of the anatomical map, and render to the display the multiple IEGM traces representing electrical activity in the tissue that is sensed by respective ones of the catheter electrodes responsively to the respective ones of the catheter electrodes being located in proximity to the selected region of the anatomical map, and the respective quality of contact of the respective ones of the catheter electrodes with the tissue of the heart at the respective locations.

Additionally in accordance with an embodiment of the present disclosure the processing circuitry configured to move a position of the at least one IEGM trace rendered on the display to follow the movement in the position of the distal end of the catheter.

Moreover in accordance with an embodiment of the present disclosure the processing circuitry configured to execute a first software program configured to compute the position of the distal end of the catheter responsively to the at least one position signal, and render to the display the anatomical map of the chamber of the heart and the representation of the distal end of the catheter, and output data indicative of the at least one IEGM trace representing electrical activity in the tissue that is sensed by the at least one of the catheter electrodes, and a second software program configured to receive the data output by the first software program, and render to the display over the at least part of the anatomical map, the at least one IEGM.

Further in accordance with an embodiment of the present disclosure the processing circuitry is configured to render the at least one IEGM trace as a progressing IEGM trace representing current electrical activity in the tissue.

Still further in accordance with an embodiment of the present disclosure the processing circuitry is configured to render the at least one IEGM trace as a static IEGM trace representing recorded electrical activity in the tissue.

Additionally in accordance with an embodiment of the present disclosure the processing circuitry is configured to render the at least one IEGM trace as a progressing IEGM trace representing recorded electrical activity in the tissue.

Moreover, in accordance with an embodiment of the present disclosure the processing circuitry is configured to receive a user input selecting a point on the anatomical map, and render to the display the at least one IEGM trace responsively to the user input.

There is also provided in accordance with another embodiment of the present disclosure, a medical system, including a catheter configured to be inserted into a chamber of a heart of a living subject, and including a distal end including catheter electrodes configured to contact tissue at respective locations within the chamber of the heart, at least one position sensor configured to provide at least one position signal indicative of a position of the distal end, a display, and processing circuitry configured to compute the position of the distal end of the catheter responsively to the at least one position signal, assess a respective quality of contact of each of at least a sub-set of the catheter electrodes with the tissue, render to the display a three-dimensional (3D) anatomical map of the chamber of the heart and a 3D representation of the distal end of the catheter, and render to the display multiple intracardiac electrogram (IEGM) traces representing electrical activity in the tissue that is sensed by respective ones of the catheter electrodes responsively to the respective quality of contact of each of the respective ones of the catheter electrodes with the tissue of the heart at the respective locations.

Further in accordance with a non-claimed embodiment of the present disclosure the processing circuitry is configured to render to the display the multiple IEGM traces representing electrical activity in the tissue that is sensed by respective ones of the catheter electrodes responsively to the respective quality of contact of each of the respective ones of the catheter electrodes with the tissue of the heart at the respective locations exceeding a threshold quality of contact.

Still further in accordance with an embodiment of the present disclosure the processing circuitry is configured to render to the display the multiple IEGM traces representing electrical activity in the tissue that is sensed by n respective ones of the catheter electrodes responsively to the respective quality of contact of each of the n respective ones of the catheter electrodes with the tissue of the heart being among n highest qualities of contact of the at least sub-set of the catheter electrodes.

Additionally in accordance with an embodiment of the present disclosure the processing circuitry is configured to receive a user input selecting the sub-set of the catheter electrodes.

Moreover in accordance with an embodiment of the present disclosure the processing circuitry is configured to receive a user input selecting a region of the anatomical map, and render to the display the multiple IEGM traces representing electrical activity in the tissue that is sensed by respective ones of the catheter electrodes responsively to the respective ones of the catheter electrodes being located in proximity to the selected region of the anatomical map, and the respective quality of contact of the respective ones of the catheter electrodes with the tissue of the heart at the respective locations.

There is also provided in accordance with still another embodiment of the present disclosure, a medical method, including providing at least one position signal indicative of a position of a distal end of a catheter inserted into a chamber of a heart of a living subject, computing the position of the distal end of the catheter responsively to the at least one position signal, rendering to a display a three-dimensional (3D) anatomical map of the chamber of the heart and a 3D representation of the distal end of the catheter, and rendering to the display over at least part of the anatomical map, at least one intracardiac electrogram (IEGM) trace representing electrical activity in tissue of the chamber that is sensed by at least one catheter electrode of the catheter.

There is also provided in accordance with still another embodiment of the present disclosure, a medical method, including providing at least one position signal indicative of a position of a distal end of a catheter inserted into a chamber of a heart of a living subject, computing the position of the distal end of the catheter responsively to the at least one position signal, assessing a respective quality of contact of each of at least a sub-set of catheter electrodes of the catheter with tissue of the chamber, rendering to a display a three-dimensional (3D) anatomical map of the chamber of the heart and a 3D representation of the distal end of the catheter, and rendering to the display multiple intracardiac electrogram (IEGM) traces representing electrical activity in the tissue that is sensed by respective ones of the catheter electrodes responsively to the respective quality of contact of each of the respective ones of the catheter electrodes with the tissue of the heart at respective locations.

### BRIEF DESCRIPTION OF THE DRAWINGS

The present invention will be understood from the following detailed description, taken in conjunction with the drawings in which:
Fig. 1 is a schematic, pictorial illustration of a catheter tracking system constructed and operative in accordance with an embodiment of the present invention;
Fig. 2 is a flowchart including steps in a first method of operation of the system of Fig. 1;
Fig. 3 is a schematic view of a representation of a catheter in an anatomical map rendered in the system of Fig. 1;
Fig. 4 is a flowchart including steps in a second method of operation of the system of Fig. 1; and
Fig. 5 is a schematic view of the representation of the catheter in the anatomical map of Fig. 3 with multiple electrograms overlaying the map.

### DESCRIPTION OF EXAMPLE EMBODIMENTS

### OVERVIEW

As mentioned previously, in a two-step procedure, mapping followed by ablation, electrical activity at points within the heart is typically sensed and measured by advancing a catheter containing one or more electrodes into the heart, and acquiring data at a multiplicity of points. These data are then utilized to select the target areas at which the ablation is to be performed.

In particular, the electrical activity is typically displayed as intracardial electrogram (IEGM) traces for analysis by a physician in order to find sources of arrhythmia. A catheter electrode, which is not in contact with tissue in the heart, generally measures some electrical signal from the heart tissue and a far field signal. When the catheter electrode is in contact with the heart tissue, the amplitude of the signal is mainly based on tissue conductivity, while the far field is minor. Therefore, the physician is generally interested in analyzing the IEGM traces of electrodes in contact with the tissue.

For focal catheters with one or two electrodes, a single IEGM trace is typically displayed for a physician to analyze. A physician can quickly determine based on the form of the signal whether the catheter electrode providing the signal is in contact with the tissue. However, multi-electrode catheters simultaneously capturing electrical activity from different tissue locations may provide data for a plurality of IEGM traces to be displayed at the same time on a single display. In some cases, the number of IEGM traces may be too numerous for the physician to easily determine which of the IEGM traces are provided by electrodes in contact with the tissue, and which are not.

An example of a multielectrode catheter is the Octaray^{®} catheter, with in excess of 48 electrodes, produced by Biosense Webster Inc., of Irvine, CA, USA. The Octaray includes eight deflectable arms disposed at the distal end of a shaft, with each of the deflectable arms including six electrodes. Details of such catheter can be found in US Patent No. 11058315. Some catheters may include more electrodes, for example, but not limited to, 120 electrodes.

In addition to the need to determine electrode contact during mapping discussed above, the physician performing an ablation procedure monitors the contact of electrodes with tissue as effective ablation generally requires sufficient contact between the ablation electrode(s) and the tissue. Recently, ultra-fast ablation treatment technologies such as irreversible electroporation (IRE) have been introduced into the market. The speed as well as the ease at which the treatment may be executed may be improved by helping the physician focus on the clinically relevant input during the procedure.

Embodiments of the present invention solve at least some of the above problems during a medical procedure such as a mapping or ablation procedure, by presenting a physician with one or more IEGM traces (e.g., voltage versus time graphs) of signals acquired by electrodes of a catheter, overlaying the representation of the catheter and anatomical map so that the IEGM trace(s) are visible in the region of the screen where the physician is generally concentrating. In some embodiments, the IEGM trace(s) may move on the screen to follow movement of the catheter to allow the IEGM trace(s) to stay in the region of the screen where the physician is now concentrating.

In some embodiments, a main mapping software application may process signals provided by the catheter and body surface electrodes and provide other mapping functions such a generating and rendering the anatomical map and the representation of the catheter to a display. A secondary software application may receive data (for example, a position of the distal end of the catheter on the display, and IEGMs captured by the catheter electrodes) from the main mapping software application. The secondary software application may then display the IEGMs overlaying the anatomical map, typically centered around the distal end of the catheter. In this manner, the main mapping software application does not need to be changed, or undergoes minor changes, while other functionality is provided by the secondary software application. In other embodiments, the functionality provided herein may be implemented with a single software application.

In some embodiments, instead of showing all the IEGM traces of signals acquired by all the electrodes of the catheter, the number of IEGM traces displayed may be reduced to select one or more of the IEGM traces more relevant to the physician.

In some embodiments, the IEGM traces selected for display may be captured by groups of electrodes selected by the physician. For example, the physician may be more interested in certain groups of electrodes, such as electrodes at the distal tip of the catheter or electrodes around an equator of a basket or balloon distal end of the catheter. In some embodiments, the IEGM traces selected for display may be those electrodes in sufficient contact with tissue of a given region of the heart. The region of the heart may be a named region, which is selected by the physician (e.g., pulmonary vein, right atrium, left atrium, right ventricle, left ventricle), or a region selected on the anatomical map using a pointing device such as a mouse, stylus, or a finger.

Additionally, or alternatively, the number of IEGM traces displayed may be limited based on a quality of contact of the respective electrodes with tissue of the heart. For example, IEGM traces of electrodes having a sufficient quality of contact greater than a threshold quality of contact may be displayed, or the IEGM traces of the n electrodes having the highest quality of contact among the electrodes may be displayed.

In the above discussion, sufficiency of tissue contact is used to decide whether or not to display the IEGM traces. A quality of contact may be assessed based on different methods including impedance measurements, force or pressure measurements, or from analysis of IEGM traces, as will now be described in more detail.

The displayed may be current IEGM traces being currently captured by the electrodes or recorded IEGM traces which may be displayed as progressing IEGMs (in which different time windows of electrical activity of the tissue is shown over time) or static IEGMs (in which one or more time-windows of electrical activity of the tissue is shown without changing the IEGM data being displayed).

In response to signals provided by the catheter, processing circuitry assesses the respective quality of contact of each of the catheter electrodes with the tissue in the heart. Any one of the catheter electrodes may be in full or partial contact with the tissue of the heart. In some cases, any one of the catheter electrodes may be in contact with the tissue via another fluid such as blood of various thicknesses. The quality of contact (full or partial contact, or contact via another liquid) of any one of the catheter electrodes with the tissue may be assessed based on the signals provided by the catheter.

The term "quality of contact" as used in the specification and claims is defined herein as a quantitative indicator of the degree of contact between one of the catheter electrodes and the tissue. The "quality of contact" may be expressed directly, for example in terms a measured electrical impedance, or indirectly, for example in terms of contact force, pressure or IEGM amplitude, as will now be described below in more detail. In some embodiments, the "quality of contact" may be expressed as a measurement from the respective electrode to the tissue based on computing the position of the electrode as located in the generated anatomical map and the known location of the tissue given by the generated anatomical map.

In some embodiments, the catheter may provide signals which provide an indication of impedance between the catheter electrodes and body surface electrodes. The indication of the impedance provides an indication of a quality of contact, such that a higher value of impedance between one of the catheter electrodes and the body surface electrodes indicates a higher quality of contact between that catheter electrode and the tissue. A value of impedance may be selected to define a minimum quality of contact considered to represent sufficient contact between any one of the catheter electrodes and the tissue.

In some embodiments, the impedance between one of the catheter electrodes and another one of the electrodes on the catheter may be used as a measure of quality of contact. As disclosed in the '529 patent mentioned in the background section above, it is generally known that impedance through blood is generally lower than impedance through tissue. Accordingly, tissue contact may be assessed by comparing impedance values across a set of electrodes to premeasured impedance values when an electrode is known to be in sufficient contact with tissue and when it is known to be in contact only with blood.

In some embodiments, the method of US Patent 9168004 to Gliner, at al. may be used to assess quality of contact using a machine learning based method.

In some embodiments, the catheter may provide signals from force or pressure sensors. The indication of force or pressure provides an indication of a quality of contact, such that a higher value of force or pressure indicates a higher quality of contact between a catheter electrode and the tissue. A value of force or pressure may be selected to define a minimum quality of contact considered to represent sufficient contact between any one of the catheter electrodes and the tissue.

In some embodiments, the generated IEGM traces may be used to assess the quality of contact between any one of the catheter electrodes and the tissue. The maximum amplitude of the IEGM trace associated with one of the catheter electrodes is indicative of the quality of contact between that catheter electrode and the tissue, such that a higher value of the maximum amplitude of the IEGM trace indicates a higher quality of contact between that catheter electrode and the tissue. An amplitude value of the IEGM trace may be selected to define a minimum quality of contact considered to represent sufficient contact between any one of the catheter electrodes and the tissue.

### SYSTEM DESCRIPTION

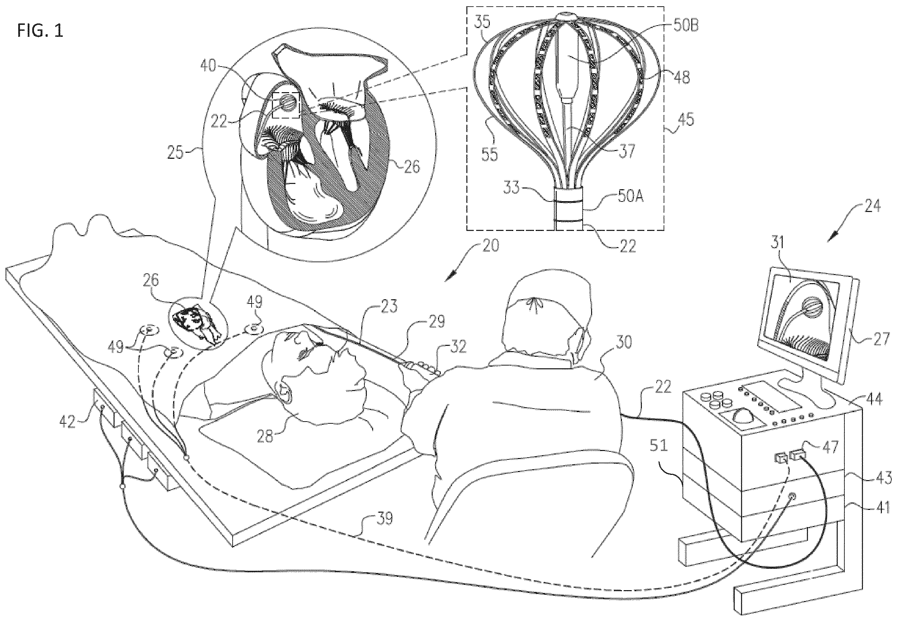

Reference is now made to Fig. 1, which is a schematic, pictorial illustration of a catheter tracking system 20, in accordance with an embodiment of the present invention. The system 20 includes a catheter 40 configured to be inserted into a body part (e.g., a chamber of a heart 26) of a living subject (e.g., a patient 28) seen in detail in inset 25. A physician 30 navigates the catheter 40 (for example, a basket catheter produced by Biosense Webster, Inc. of Irvine, CA, USA), seen in detail in inset 45, to a target location in the heart 26 of the patient 28, by manipulating a deflectable segment of an insertion tube 22 of the catheter 40, using a manipulator 32 near a proximal end 29 of the insertion tube 22, and/or deflection from a sheath 23. In the pictured embodiment, physician 30 uses catheter 40 to perform anatomical mapping of a cardiac chamber.

The catheter 40 includes a distal end 33. The distal end 33 of the catheter 40 includes an assembly 35 (e.g., a basket assembly as shown in Fig. 1 or a balloon assembly or any suitable distal end assembly, e.g., grid, flexible splines or a focal catheter) on which at least one (e.g., multiple) catheter electrode(s) 48 (only some labeled for the sake of simplicity) are disposed. The electrodes 48 are configured to contact tissue at respective locations with the chamber of the heart. The assembly 35 is disposed distally to the insertion tube 22 and may be connected to the insertion tube 22 via a coupling member of the insertion tube 22 at the distal end 33. The coupling member of the insertion tube 22 may be formed as an integral part of the rest of the insertion tube 22 or as a separate element which connects with the rest of the insertion tube 22.

The assembly 35 further comprises multiple flexible strips 55 (only two labeled for the sake of simplicity), to each of which are coupled the electrodes 48. The assembly 35 may include any suitable number of electrodes 48. In some embodiments, the assembly 35 may include ten flexible strips 55 and 120 electrodes, with twelve electrodes disposed on each flexible strip 55.

The catheter 40 includes a pusher 37. The pusher 37 is typically a tube that is disposed in a lumen of the insertion tube 22 and spans from the proximal end 29 to the distal end 33 of the insertion tube 22. A distal end of the pusher 37 is connected to distal ends of the flexible strips 55, typically via a coupling member of the pusher 37. The coupling member of the pusher 37 may be formed as an integral part of the rest of the pusher 37 or as a separate element which connects with the rest of the pusher 37. The distal end of the insertion tube 22 is connected to proximal ends of the flexible strips 55, typically via the coupling member of the distal end 33. The pusher 37 is generally controlled via the manipulator 32 to deploy the assembly 35 and change an ellipticity of the assembly 35 according to the longitudinal displacement of the pusher 37 with respect to the insertion tube 22. The actual basket assembly 35 structure may vary. For example, flexible strips 55 may be made of a printed circuit board (PCB), or of a shape-memory alloy, or any suitable material.

Embodiments described herein refer mainly to a basket distal-end assembly 35, purely by way of example. **In** alternative embodiments, the disclosed techniques can be used with a catheter having a balloon-based distal-end assembly or of any other suitable type of distal-end assembly.

Catheter 40 is inserted in a folded configuration, through sheath 23, and only after the catheter 40 exits sheath 23 is catheter 40 able to change shape by retracting pusher 37. By containing catheter 40 in a folded configuration, sheath 23 also serves to minimize vascular trauma on its way to the target location.

The distal end 33 of the catheter 40 comprises magnetic coil sensors 50A and 50B. The magnetic coil sensor 50A is shown in inset 45 at the distal edge of insertion tube 22 (i.e., at the proximal edge of basket assembly 35). The sensor 50A may be a Single-Axis Sensor (SAS), or a Double-Axis Sensor (DAS) or a Triple-Axis Sensor (TAS). Similarly, the sensor 50B may be a SAS, DAS, or TAS. Magnetic coil sensors 50A and 50B and electrodes 48 are connected by wires running through insertion tube 22 to various driver circuitries in a console 24.

**In** some embodiments, system 20 comprises a magnetic-sensing subsystem to estimate an ellipticity of the basket assembly 35 of catheter 40, as well as its elongation/retraction state, inside a cardiac chamber of heart 26 by estimating the elongation of the basket assembly 35 from the distance between sensors 50A and 50B. Patient 28 is placed in a magnetic field generated by a pad containing multiple magnetic field generator coils 42, which are driven by a unit 43. The magnetic field generator coils 42 are configured to generate respective alternating magnetic fields, having respective different frequencies, into a region where a body-part (e.g., the heart 26) of a living subject (e.g., the patient 28) is located. The magnetic coil sensors 50A and 50B are configured to output electrical signals responsively to detecting the respective magnetic fields. For example, if there are nine magnetic field generator coils 42 generating nine respective different alternating magnetic fields with nine respective different frequencies, the electrical signals output by the magnetic coil sensors 50 will include components of the nine different frequency alternating magnetic fields. The magnitude of each of the magnetic fields varies with distance from the respective magnetic field generator coils 42 such that the location of the magnetic coil sensors 50 may be determined from the magnetic fields sensed by the magnetic coil sensors 50. Therefore, the transmitted alternating magnetic fields generate the electrical signals in sensors 50A and 50B, so that the electrical signals are indicative of position and orientation of the magnetic coil sensors 50 in the heart chamber.

The generated signals are transmitted to console 24 and become corresponding electrical inputs to processing circuitry 41. The processing circuitry 41 may use the signals to compute: the elongation of the basket assembly 35, in order to estimate basket ellipticity and elongation/retraction state from the calculated distance between sensors 50A and 50B; and compute a relative orientation between the axes of the sensors 50A and 50B to estimate a shape of the expandable distal end assembly 35 (e.g., a basket shape) responsively to the relative orientation, as described in more detail below.

The bow of the flexible strips 55 and/or the positions of the electrodes 48 (or other features) on the flexible strips 55 with respect to a fixed point on the catheter 40 (such as the distal tip of the insertion tube 22) may be measured for various distances between the magnetic sensors 50A, 50B and for various relative orientation angles between the magnetic sensors 50A, 50B. For example, the positions of the electrodes 48 with respect to the fixed point on the catheter 40 may be measured for every 0.2 mm movement of the pusher 37 with respect to the insertion tube 22 and for every 1 degree of relative orientation between the magnetic sensors 50A, 50B (up to a maximum sideways movement of the assembly 35). At each different distance/relative-orientation combination, the computed distance and computed relative orientation angle between the magnetic sensors 50A, 50B is recorded along with the position data of the electrodes 48. This data may then be used to estimate the bow of the flexible strips 55 and/or the positions of the electrodes 48 (or other features) on the flexible strips 55 with respect to a fixed point on the catheter 40 (such as the distal tip of the insertion tube 22) responsively to the computed distance and relative orientation angle between the magnetic sensors 50A, 50B.

Additionally, or alternatively, the bow of the flexible strips 55 may be estimated based on the following assumptions: (a) each of the flexible strips 55 is of a fixed and known length; (b) each of the flexible strips 55 is connected to the pusher 37 via a coupler, with the distal ends of the flexible strips 55 being substantially perpendicular (within an error of plus or minus 10 degrees) to the longitudinal axis of the insertion tube 22; (c) each of the flexible strips 55 is connected to the insertion tube 22 via a coupler, which couples the proximal ends of the flexible strips 55 to the insertion tube 22, substantially parallel (within an error of plus or minus 10 degrees) to the longitudinal axis of the insertion tube 22. Based on the above assumptions (a)-(c), and the computed positions of the couplers based on the computed positions of the magnetic sensors 50A, 50B, the bow of each of the flexible strips 55 may be computed using a third-degree polynomial. In some embodiments, the bow of the flexible strips 55 and/or the positions of the electrodes 48 (or other features) on the flexible strips 55 with respect to a fixed point on the catheter 40 (such as the distal tip of the insertion tube 22) may be computed based on the computed distance and orientation between the magnetic sensors 50A, 50B and a model of the catheter 40 which provides the bow of the flexible strips 55 and/or the positions of the electrodes 48 for the computed distance based on the mechanical properties and dimensions of the flexible strips 55.

A method of position and/or direction sensing using external magnetic fields and magnetic coil sensors, such as sensors 50A and 50B, is implemented in various medical applications, for example, in the CARTO^{®} system, produced by Biosense-Webster, and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

In some embodiments, the processing circuitry 41 uses position-signals received from the electrodes 48 or body surface electrodes 49, and the magnetic sensor 50 to estimate a position or orientation of the assembly 35 inside a body part, such as inside a cardiac chamber. In some embodiments, the processing circuitry 41 correlates the position signals received from the electrodes 48, 49 with previously acquired magnetic location-calibrated position signals, to estimate the position of the assembly 35 inside the body part. The position coordinates of the electrodes 48 may be determined by the processing circuitry 41 based on, among other inputs, measured impedances, voltages or on proportions of currents distribution, between the electrodes 48 and the body surface electrodes 49.

The method of position sensing using current distribution measurements and/or external magnetic fields is implemented in various medical applications, for example, in the Carto^{®} system, produced by Biosense Webster Inc. (Irvine, California), and is described in detail in U.S. Patents 5,391,199, 6,690,963, 6,484,118, 6,239,724, 6,618,612, 6,332,089, 7,756,576, 7,869,865, and 7,848,787, in PCT Patent Publication WO 96/05768, and in U.S. Patent Application Publications 2002/0065455 A1, 2003/0120150 A1 and 2004/0068178 A1.

The Carto^{®}3 system applies Active Current Location (ACL) which is a hybrid current-distribution and magnetic-based position-tracking technology. In some embodiments, using ACL, the processing circuitry 41 estimates the positions of the electrodes 48. In some embodiments, the signals received from the electrodes 48, 49 are correlated with a current-to-position matrix (CPM) which maps current distribution ratios (or another electrical value) with a position that was previously acquired from magnetic location-calibrated position signals. The current distribution ratios are based on measurements of the body surface electrodes 49 of current flowing from the electrodes 48 to the body surface electrodes 49.

In some embodiments, to visualize catheters which do not include a magnetic sensor, the processing circuitry 41 may apply an electrical signal-based method, referred to as Independent Current Location (ICL) technology. In ICL, the processing circuitry 41 calculates a local scaling factor for each voxel of the volume in which catheters are visualized. The factor is determined using a catheter with multiple electrodes having a known spatial relationship, such as a lasso-shaped catheter. However, although yielding accurate local scaling (e.g., over several millimeters), ICL is less accurate when applied to a volume of a whole heart chamber, whose size is in the order of centimeters. The ICL method, in which positions are calculated based on current distribution proportions can have errors and may yield a distorted shape of the assembly 35, due to the non-linear nature of the current-based ICL space. In some embodiments, the processing circuitry 41 may apply the disclosed ICL method to scale ICL space and the assembly 35 shape into a correct one, based on known smaller scale distances between electrodes of a lasso-shaped catheter, for example, as well as based on larger scale distances, themselves based on the known distance between the electrodes 48 at the ends of the assembly 35.

Processing circuitry 41, typically part of a general-purpose computer, is further connected via a suitable front end and interface circuits 44, to receive signals from body surface-electrodes 49. Processing circuitry 41 is connected to surface-electrodes 49 by wires running through a cable 39 to the chest of patient 28. The catheter 40 includes a connector 47 disposed at the proximal end 29 of the insertion tube 22 for coupling to the processing circuitry 41.

In some embodiments, processing circuitry 41 renders to a display 27, a representation 31 of at least a part of the catheter 40 and an anatomical map or body-part, (e.g., from a mapping process or from a scan (e.g., CT or MRI) of the body-part previously registered with the system 20), responsively to computed position coordinates of the insertion tube 22 and the flexible strips 55.

Processing circuitry 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory. The system 20 may also include a memory 51 used by the processing circuitry 41.

The example illustration shown in Fig. 1 is chosen purely for the sake of conceptual clarity. Fig. 1 shows only elements related to the disclosed techniques for the sake of simplicity and clarity. System 20 typically comprises additional modules and elements that are not directly related to the disclosed techniques, and thus are intentionally omitted from Fig. 1 and from the corresponding description. The elements of system 20 and the methods described herein may be further applied, for example, to control an ablation of tissue of heart 26.

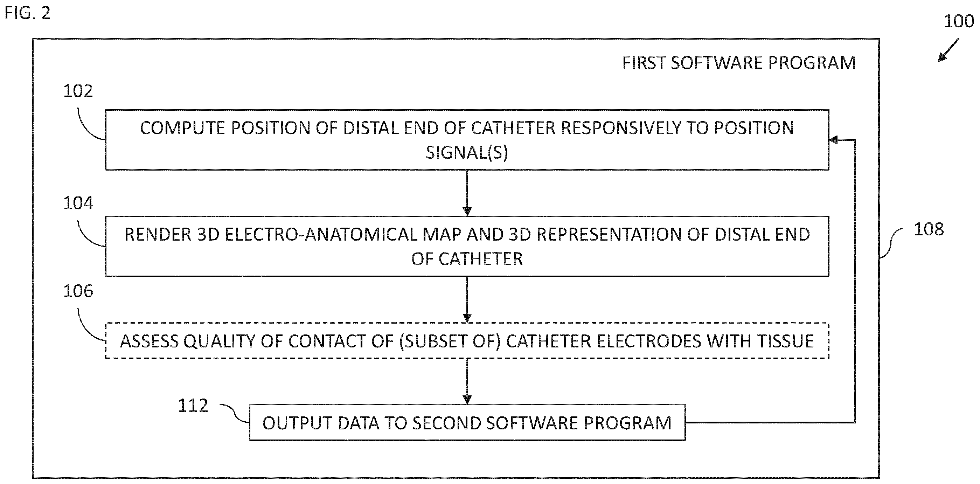

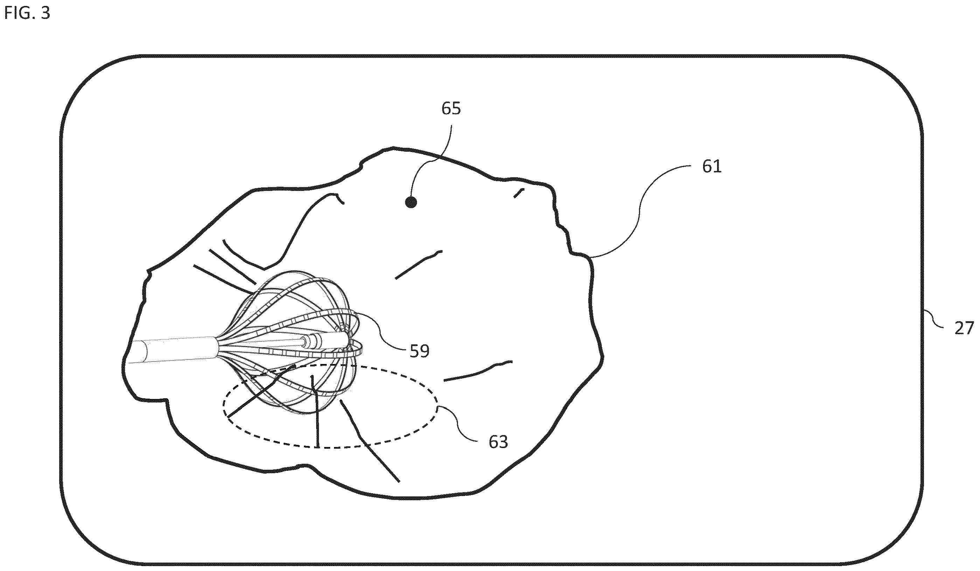

Reference is now made to Figs. 2 and 3. Fig. 2 is a flowchart 100 including steps in a first method of operation of the system 20 of Fig. 1. Fig. 3 is a schematic view of a representation 59 of the catheter 40 in an anatomical map 61 rendered in the system 20 of Fig. 1.

As previously described with reference to Fig. 1, the position of the distal end of the catheter 40 inside a chamber of the heart may be found using a variety of methods, for example, based on signals provided by at least one position sensor, which may include the magnetic coil sensors 50 and/or the electrodes 48 and/or the body surface electrodes 49. The position sensor(s) 50, 48, 49 are configured to provide at least one position signal indicative of a position or orientation of the distal end with reference to a 3D anatomical map of the chamber of the heart. In some embodiments, the position of the distal end of the catheter 40 may be found using imaging techniques such as ultrasonic scanning and suitable processing of the ultrasound images. The processing circuitry 41 is configured to compute the position of the distal end of the catheter 40 responsively to the position signal(s) provided by the position sensor(s) 50, 48, 49 (block 102). That is, circuitry 41 computes the position of the distal end of the catheter from the at least one position signal to define the position and orientation of the distal end or electrodes of the catheter with respect to a chamber of the heart and thereby render to the display a three-dimensional (3D) anatomical map of the chamber of the heart and a 3D representation of the location and orientation of the distal end of the catheter as referenced to the 3D anatomical map. By providing the 3D anatomical map and the 3D representation of the catheter, the physician is able to identify where the position of the distal end of the catheter (and its electrodes) are located with reference to the chamber of the heart and move the distal end of the catheter within the heart to specific locations in the heart chamber by viewing the 3D representations of the heart and catheter.

The processing circuitry 41 is configured to render to the display 27 the three-dimensional (3D) anatomical map 61 of the chamber of the heart 26 and the 3D representation 59 of the distal end of the catheter 40 (block 104) as positioned or orientated with respect to the 3D anatomical map. The anatomical map 61 may be generated using any suitable anatomical map generation method, for example, but not limited to, Fast Anatomical Mapping (FAM). FAM is described in US Patent 10,918,310 to Cohen, et al. In FAM, a smooth shell is generated around a three-dimensional (3D) cloud of data points, such as a cloud of computed electrode positions of the electrodes 48 so that the position and orientation of the distal end and electrodes 48 with respect to the chamber of the heart can be graphically represented in the 3D anatomical map 61 of the heart.

In some embodiments, the processing circuitry 41 is configured to receive signals from the catheter 40, and in response to the signals, assess a respective quality of contact of each of (at least a sub-set of) the catheter electrodes 48 with the tissue (block 106).

Any one of the catheter electrodes 48 may be in full or partial contact with the tissue of the heart 26. In some cases, any one of the catheter electrodes 48 may be in contact with the tissue via another fluid such as blood of various thicknesses. The quality of contact (full or partial contact, or contact via another liquid) of any one of the catheter electrodes 48 with the tissue may be assessed based on the signals provided by the catheter 40.

As previously mentioned, the term "quality of contact" is a quantitative indicator of the degree of (electrical) contact between one of the catheter electrodes 48 and the tissue. The "quality of contact" may be expressed directly, for example in terms a measured electrical impedance, or indirectly, for example in terms of contact force, pressure or IEGM amplitude, as will now be described below in more detail. The "quality of contact" may also be expressed in terms of distance from the electrodes to the tissue based on computed positions of the electrodes 48 and the anatomical map 61.

In some embodiments, the catheter 40 may provide signals which provide an indication of impedance between the catheter electrodes 48 and the body surface electrodes 49. The indication of the impedance provides an indication of a quality of contact, such that a higher value of impedance between one of the catheter electrodes 48 and the body surface electrodes 49 indicates a higher quality of contact between that catheter electrode 48 and the tissue. A value of impedance may be selected to define a minimum quality of contact considered to represent sufficient contact between any one of the catheter electrodes 48 and the tissue.

In some embodiments, the impedance between one of the catheter electrodes 48 and another one of the electrodes on the catheter 40 may be used as a measure of quality of contact. As disclosed in the '529 patent mentioned in the background section above, it is generally known that impedance through blood is generally lower than impedance through tissue. Accordingly, tissue contact may be assessed by comparing impedance values across a set of electrodes to premeasured impedance values when an electrode is known to be in sufficient contact with tissue and when it is known to be in contact only with blood.

In some embodiments, the method of US Patent 9168004 to Gliner, at al. may be used to assess quality of contact. The '004 Patent describes using machine learning to determine catheter electrode contact. The '004 Patent describes cardiac catheterization being carried out by memorizing a designation of a contact state between an electrode of the probe and the heart wall as an in-contact state or an out-of-contact state, and making a series of determinations of an impedance phase angle of an electrical current passing through the electrode and another electrode, identifying maximum and minimum phase angles in the series, and defining a binary classifier adaptively as midway between the extremes. A test value is compared to the classifier as adjusted by a hysteresis factor, and a change in the contact state is reported when the test value exceeds or falls below the adjusted classifier.

In some embodiments, the catheter 40 may provide signals from force or pressure sensors (not shown), disposed at different locations on the flexible strips 55, that provide an indication of force or pressure exerted by the catheter electrodes 48 on the tissue. The indication of force or pressure provides an indication of a quality of contact, such that a higher value of force or pressure indicates a higher quality of contact between that catheter electrode 48 and the tissue. A value of force or pressure may be selected to define a minimum quality of contact considered to represent sufficient contact between any one of the catheter electrodes 48 and the tissue. These embodiments, may use any suitable force or pressure sensors as well as any suitable method for measuring the force or pressure, including any of the Patents or Patent Publications mentioned in the background section including the method described in US Patent Publication 2013/0085416 of Mest and describes a method for the in vivo re-calibration of a force sensing probe such as an electrophysiology catheter which provides for the generation of an auto zero zone. The distal tip of the catheter or other probe is placed in a body cavity within the patient. Verification that there is no tissue contact is made using electrocardiogram (ECG) or impedance data, fluoroscopy or other real-time imaging data and/or an anatomical mapping system. Once verification that there is no tissue contact made, the system recalibrates the signal emanating from the force sensor setting it to correspond to a force reading of zero grams and this recalibrated baseline reading is used to generate and display force readings based on force sensor data.

In some embodiments, intracardiac electrogram (IEGM) traces generated by the processing circuitry 41 may be used to assess the quality of contact between any one of the catheter electrodes 48 and the tissue. The maximum amplitude of the IEGM trace associated with one of the catheter electrodes 48 is indicative of the quality of contact between that catheter electrode 48 and the tissue, such that a higher value of the maximum amplitude of the IEGM trace indicates a higher quality of contact between that catheter electrode 48 and the tissue. An amplitude value of the IEGM trace may be selected to define a minimum quality of contact considered to represent sufficient contact between any one of the catheter electrodes 48 and the tissue.

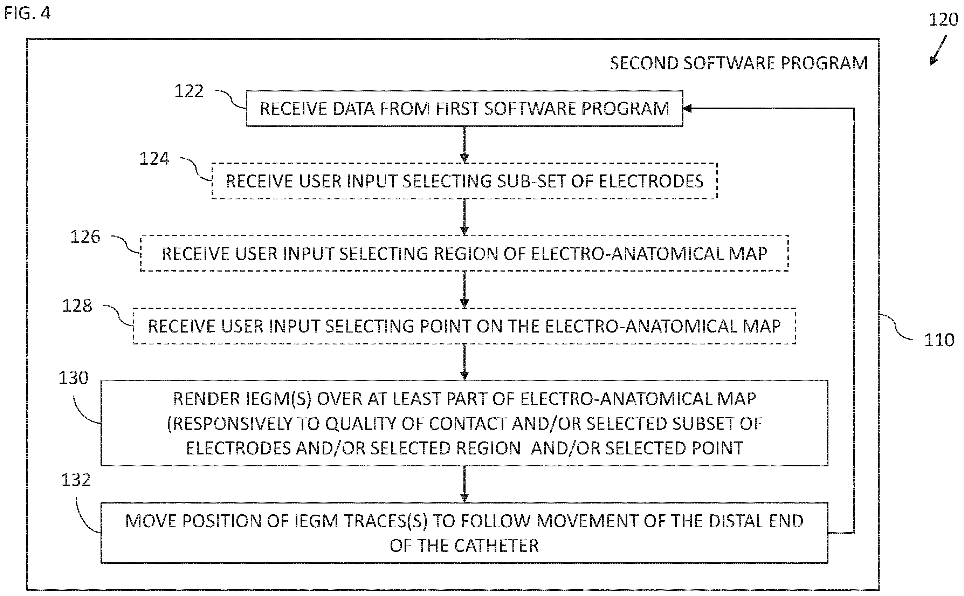

In some embodiments, the steps of the methods of Figs. 2 and 4 are executed via a single software program. In some embodiments, the method described with reference to Fig. 2 is executed by a first software program and the method described with reference to Fig. 4 is executed by a second software program.

In some embodiments, the processing circuitry 41 is configured to execute a main mapping software application 108 (also referred herein as a "first software program") to perform the steps of blocks 102-106, while a secondary software application 110 (Fig. 4) (also referred herein as a "second software program) may receive data (for example, a position of the distal end of the catheter 40 on the display 27, and IEGMs of the catheter electrodes 48) from the main mapping software application. The steps performed by the secondary software application are described with reference to Fig. 4. In this manner, the main mapping software application does not need to be changed, or undergoes minor changes, while other functionality is provided by the secondary software application. In other embodiments, the steps described with reference to Figs. 2 and 4 may be implemented with a single software application.

Therefore, in some embodiments, the main mapping software application 108 running on the processing circuitry 41 is configured to output data indicative of the IEGM trace(s) representing electrical activity in the tissue that is sensed by the catheter electrode(s) 48, and a position of the distal end of the catheter 40 on the display 27 (block 112), for example using a TCP-based communication protocol.

Reference is now made to Fig. 4, which is a flowchart 120 including steps in a second method of operation of the system 20 of Fig. 1. Reference is also made to Fig. 3.

As previously mentioned, the steps described with reference to Fig. 4 may be performed by the secondary software application 110. Therefore, the processing circuitry 41 is configured to execute the secondary software application 110 to receive the data output by the main mapping software application 108 (block 122), for example using a TCP-based communication protocol. In other embodiments, the steps described with reference to Figs. 2 and 4 may be implemented with a single software application.

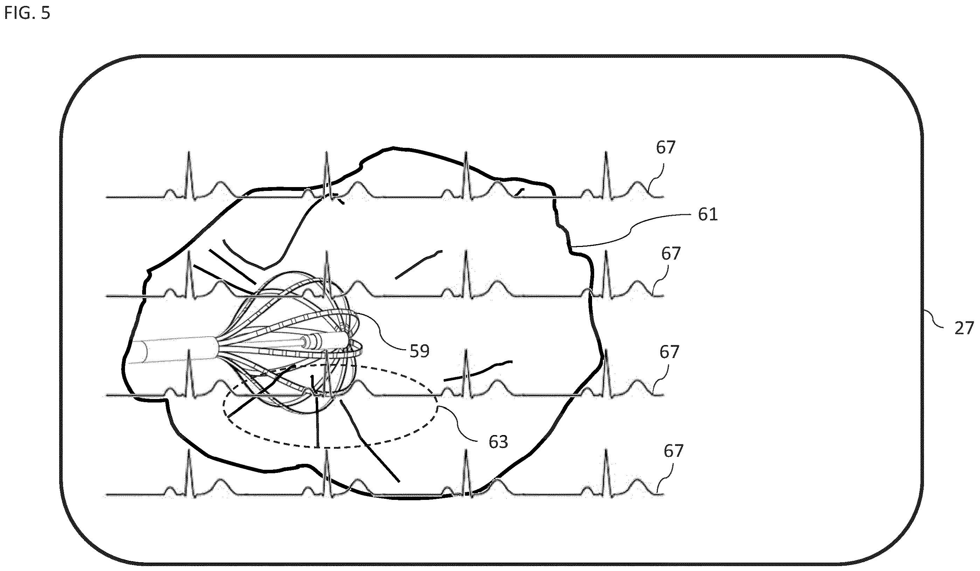

The physician 30 is able to select a region of, and/or a point on, the anatomical map 61, and/or a sub-set of the electrodes 48 as criteria in rendering one or more IEGMs to the display 27, as described in more detail below with reference to Fig. 5. In some embodiments, the processing circuitry 41 is configured to receive a user input selecting a sub-set of the catheter electrodes (block 124). The sub-set may be selected from predefined sub-sets. For example, one sub-set may include electrodes at the distal tip of a basket-shaped catheter or electrodes around an equator of a basket or balloon-shaped catheter. In some embodiments, the processing circuitry 41 is configured to receive a user input selecting a region 63 of the anatomical map 61 (block 126). The region may be selected from a predefined list of regions, such as pulmonary vein, right atrium, left atrium, right ventricle, left ventricle, or the region 63 may be selected by delineating the region on the anatomical map 61 using a pointing device such as a mouse, stylus, or finger, as shown in Fig. 3. In some embodiments, the processing circuitry 41 is configured to receive a user input selecting a point 65 on the anatomical map 61 (block 128).

Reference is now made to Fig. 5, which is a schematic view of the representation 59 of the catheter 40 in the anatomical map 61 of Fig. 3 with multiple intracardiac electrograms (IEGM) traces 67 overlaying the map 61. Reference is also made to Fig. 4.

The processing circuitry 41 is configured to render to the display 27, over at least part of the anatomical map 61 (and/or over at least part of the representation 59 of the catheter 40), one or more IEGM traces 67 representing electrical activity in the tissue of the heart 26 that is sensed by one or more of the catheter electrodes 48 (block 130). For example, if the physician 30 selected the point 65 in the step of block 128, the processing circuitry 41 may be configured to render to the display one IEGM trace 67 which represents electrical activity closest to the point 65. The rendered IEGM trace 67 may represent current electrical activity captured by one of the electrodes 48 at, or near, the point 65, or previously captured electrical activity at, or near, the point 65. Therefore, in some embodiments, the processing circuitry 41 is configured to render one IEGM trace 67 at least partially over the anatomical map 61 responsively to receiving the user input selecting the point 65 on the anatomical map 61.

In some embodiments, the processing circuitry 41 is configured to render to the display 27 over at least part of the anatomical map 61, multiple IEGM traces 67 representing electrical activity in the tissue of the heart 26 that is sensed by respective catheter electrodes 48.

The number of IEGM traces 67 rendered to the display 27 may be dependent on the quality of contact of each electrode 48 or a sub-set of electrodes 48 (for example, the sub-set of electrodes 48 selected in the step of block 124) so that the more relevant IEGM traces 67 are rendered to the display 27. Therefore, in some embodiments, the processing circuitry 41 is configured to render to the display 27 (over at least part of the anatomical map 61) multiple IEGM traces 67 representing electrical activity in the tissue that is sensed by respective catheter electrodes 48 responsively to the respective quality of contact of each respective catheter electrode 48 with the tissue of the heart at the respective locations. In some embodiments, the processing circuitry 41 is configured to render to the display 27 (over at least part of the anatomical map 61) the multiple IEGM traces 67 representing electrical activity in the tissue that is sensed by respective catheter electrodes 48 responsively to the respective quality of contact of each respective catheter electrode 48 with the tissue of the heart at the respective locations exceeding a threshold quality of contact. In other words, the IEGM traces 67 of the electrodes 48 (or the selected sub-set of the electrodes 48) having a quality of contact exceeding the threshold quality of contact are rendered to the display 27 over at least part of the anatomical map 61 by the processing circuitry 41.

The number of IEGM traces 67 rendered to display 27 may be limited to n IEGM traces 67 so that the IEGM traces 67 of n corresponding electrodes 48 (or n electrodes 48 of the selected sub-set of the electrodes 48) closest to the tissue (measured by the quality of contact) are rendered to the display 27. Therefore, in some embodiments, the processing circuitry 41 is configured to render to the display 27 n IEGM traces 67 representing electrical activity in the tissue that is sensed by n respective catheter electrodes 48 (e.g., n electrodes 48 from all the electrodes 48 or of the selected sub-set of electrodes 48) responsively to the respective quality of contact of each of the n respective catheter electrodes 48 with the tissue of the heart being among n highest qualities of contact of the electrodes 48 or the sub-set of the catheter electrodes 48. The value n may be set to any suitable number by the physician 30. The value n may depend on the number of electrodes 48 and/or the size of the display 27.

In some embodiments, the IEGM traces 67 may be limited to the IEGM traces 67 captured by respective electrodes 48 from one or more selected regions 63 of the heart 26. Therefore, in some embodiments, the processing circuitry 41 is configured to render to the display 27 multiple IEGM traces 67 representing electrical activity in the tissue that is sensed by respective catheter electrodes 48 responsively to: the respective catheter electrodes 48 being located in proximity to the selected region 63 of the anatomical map 61; and optionally the respective quality of contact of the respective catheter electrodes 48 with the tissue of the heart 26 at the respective locations (exceeding a threshold quality of contact).

The IEGM traces 67 may be progressing signals (i.e., dynamically change with time) or static signals of current, or previously recorded, electrical activity captured from the tissue of the heart 26. Therefore, in some embodiments, the processing circuitry 41 is configured to render the IEGM trace(s) 67 as progressing IEGM trace(s) representing current electrical activity in the tissue. In some embodiments, the processing circuitry 41 is configured to render the IEGM trace(s) 67 as static IEGM trace(s) representing recorded electrical activity in the tissue. In some embodiments, the processing circuitry 41 is configured to render the IEGM trace(s) 67 as progressing IEGM trace(s) representing recorded electrical activity in the tissue.

In some embodiments, the processing circuitry 41 is configured to move a position of the IEGM trace(s) 67 rendered on the display 27 to follow movement in the position of the distal end of the catheter 40 (block 132), as provided by the step of block 102 of Fig. 2.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 72% to 108%.

Various features of the invention which are, for clarity, described in the contexts of separate embodiments may also be provided in combination in a single embodiment, where in accordance with the scope of the appended claims. Conversely, various features of the invention which are, for brevity, described in the context of a single embodiment may also be provided separately or in any suitable sub-combination, where in accordance with the scope of the appended claims.

The embodiments described above are cited by way of example, and the present invention is not limited by what has been particularly shown and described hereinabove. Rather the scope of the invention is defined by the appended claims.

## Claims

1. A medical system (20), comprising:
a catheter (40) configured to be inserted into a chamber of a heart of a living subject, and including a distal end (33) comprising catheter electrodes (48) configured to contact tissue at respective locations within the chamber of the heart;
at least one position sensor (50A, 50B) configured to provide at least one position signal indicative of a position of the distal end;
a display (27); and
processing circuitry (41) configured to:
compute the position of the distal end of the catheter responsively to the at least one position signal;
assess a respective quality of contact of each of at least a sub-set of the catheter electrodes with the tissue;
render to the display a three-dimensional (3D) anatomical map of the chamber of the heart and a 3D representation of the distal end of the catheter;
select multiple intracardiac electrogram (IEGM) traces (67), representing electrical activity in the tissue that is sensed by respective ones of the catheter electrodes, to be rendered to the display, responsively to the respective quality of contact of each of the respective ones of the catheter electrodes with the tissue of the heart at the respective locations; and
render to the display the selected multiple intracardiac electrogram (IEGM) traces (67),
wherein the processing circuitry is configured to select the multiple IEGM traces representing electrical activity in the tissue that is sensed by n respective ones of the catheter electrodes responsively to the respective quality of contact of each of the n respective ones of the catheter electrodes with the tissue of the heart being among n highest qualities of contact of the at least sub-set of the catheter electrodes.

2. The system according to claim 1, wherein the processing circuitry is configured to receive a user input selecting the sub-set of the catheter electrodes.

3. The system according to claim 1, wherein the processing circuitry is configured to:
receive a user input selecting a region of the anatomical map; and
render to the display the selected multiple IEGM traces representing electrical activity in the tissue that is sensed by respective ones of the catheter electrodes responsively to: the respective ones of the catheter electrodes being located in proximity to the selected region of the anatomical map; and the respective quality of contact of the respective ones of the catheter electrodes with the tissue of the heart at the respective locations.

4. The medical system of claim 1, wherein the processing circuitry is configured to render the selected multiple intracardiac electrogram (IEGM) traces (67) to the display over at least part of the anatomical map.

5. The system according to claim 1, wherein the processing circuitry configured to move a position of the at least one IEGM trace rendered on the display to follow the movement in the position of the distal end of the catheter.

6. The system according to claim 1, wherein the processing circuitry configured to execute:
a first software program configured to:
compute the position of the distal end of the catheter responsively to the at least one position signal; and
render to the display the anatomical map of the chamber of the heart and the representation of the distal end of the catheter; and
output data indicative of the at least one IEGM trace representing electrical activity in the tissue that is sensed by the at least one of the catheter electrodes; and
a second software program configured to:
receive the data output by the first software program; and
render to the display over the at least part of the anatomical map, the at least one IEGM.

7. The system according to claim 1, wherein the processing circuitry is configured to render the at least one IEGM trace (i) as a progressing IEGM trace representing current electrical activity in the tissue, (ii) as a static IEGM trace representing recorded electrical activity in the tissue, or (iii) as a progressing IEGM trace representing recorded electrical activity in the tissue.

8. The system according to claim 1, wherein the processing circuitry is configured to:
receive a user input selecting a point on the anatomical map; and
render to the display the selected multiple IEGM traces responsively to the user input.

9. A computer-implemented medical method, comprising:
receiving at least one position signal indicative of a position of a distal end of a catheter inserted into a chamber of a heart of a living subject;
computing the position of the distal end of the catheter responsively to the at least one position signal;
assessing a respective quality of contact of each of at least a sub-set of catheter electrodes of the catheter with tissue of the chamber;
rendering to a display a three-dimensional (3D) anatomical map of the chamber of the heart and a 3D representation of the distal end of the catheter;
selecting multiple intracardiac electrogram (IEGM) traces (67), representing electrical activity in the tissue that is sensed by respective ones of the catheter electrodes, to be rendered to the display, responsively to the respective quality of contact of each of the respective ones of the catheter electrodes with the tissue of the heart at respective locations; and
rendering to the display the selected multiple intracardiac electrogram (IEGM) traces,
wherein selecting the multiple IEGM traces comprises selecting the multiple IEGM traces representing electrical activity in the tissue that is sensed by n respective ones of the catheter electrodes responsively to the respective quality of contact of each of the n respective ones of the catheter electrodes with the tissue of the heart being among n highest qualities of contact of the at least sub-set of the catheter electrodes.

10. The medical method of claim 9, comprising rendering the selected multiple intracardiac electrogram (IEGM) traces to the display over at least part of the anatomical map.

## Patentansprüche

1. Medizinisches System (20), umfassend:
einen Katheter (40), der konfiguriert ist, um in eine Kammer eines Herzens eines lebenden Subjekts eingeführt zu werden, und ein distales Ende (33), umfassend Katheterelektroden (48), einschließt, die konfiguriert sind, um Gewebe an jeweiligen Stellen innerhalb der Kammer des Herzens zu berühren;
mindestens einen Positionssensor (50A, 50B), der konfiguriert ist, um mindestens ein Positionssignal bereitzustellen, das eine Position des distalen Endes angibt;
eine Anzeige (27); und
eine Verarbeitungsschaltung (41), die konfiguriert ist zum:
Berechnen der Position des distalen Endes des Katheters als Reaktion auf das mindestens eine Positionssignal;
Bewerten einer jeweiligen Berührungsqualität von jeder von mindestens einer Teilmenge der Katheterelektroden mit dem Gewebe;
Rendern einer dreidimensionalen (3D) anatomischen Karte der Herzkammer und einer 3D-Darstellung des distalen Endes des Katheters auf die Anzeige;
Auswählen multipler intrakardialer Elektrogramm-Kurven (IEGM-Kurven) (67), die eine elektrische Aktivität in dem Gewebe darstellen, die durch jeweilige der Katheterelektroden erfasst wird, um auf die Anzeige gerendert zu werden, als Reaktion auf die jeweilige Berührungsqualität jeder der jeweiligen der Katheterelektroden mit dem Herzgewebe an den jeweiligen Stellen; und
Rendern der ausgewählten multiplen intrakardialen Elektrogramm-Kurven (IEGM-Kurven) (67) auf die Anzeige,
wobei die Verarbeitungsschaltung konfiguriert ist, um die multiplen IEGM-Kurven auszuwählen, die die elektrische Aktivität in dem Gewebe darstellen, die durch n jeweilige der Katheterelektroden erfasst wird, als Reaktion auf die jeweilige Berührungsqualität jeder der n jeweiligen der Katheterelektroden mit dem Herzgewebe, die zu den n höchsten Berührungsqualitäten der mindestens einen Teilmenge der Katheterelektroden gehört.

2. System nach Anspruch 1, wobei die Verarbeitungsschaltlogik konfiguriert ist, um eine Benutzereingabe zu empfangen, die die Teilmenge der Katheterelektroden auswählt.

3. System nach Anspruch 1, wobei die Verarbeitungsschaltung konfiguriert ist zum:
Empfangen einer Benutzereingabe, die einen Bereich der anatomischen Karte auswählt; und
Rendern der ausgewählten multiplen IEGM-Kurven auf die Anzeige, die die elektrische Aktivität in dem Gewebe darstellen, die durch jeweilige der Katheterelektroden erfasst wird, als Reaktion darauf, dass sich die jeweiligen der Katheterelektroden in der Nähe des ausgewählten Bereichs der anatomischen Karte befinden; und der jeweiligen Berührungsqualität der jeweiligen der Katheterelektroden mit dem Herzgewebe an den jeweiligen Stellen.

4. Medizinisches System nach Anspruch 1, wobei die Verarbeitungsschaltung konfiguriert ist, um die ausgewählten multiplen intrakardialen Elektrogramm-Kurven (IEGM-Kurven) (67) über mindestens einen Teil der anatomischen Karte auf die Anzeige zu rendern.

5. System nach Anspruch 1, wobei die Verarbeitungsschaltung konfiguriert ist, um eine Position der mindestens einen auf die Anzeige gerenderten IEGM-Kurve zu bewegen, um der Bewegung der Position des distalen Endes des Katheters zu folgen.

6. System nach Anspruch 1, wobei die Verarbeitungsschaltung konfiguriert ist zum Ausführen von:
einem ersten Softwareprogramm, das konfiguriert ist zum:
Berechnen der Position des distalen Endes des Katheters als Reaktion auf das mindestens eine Positionssignal; und
Rendern der anatomischen Karte der Herzkammer und der Darstellung des distalen Endes des Katheters auf die Anzeige; und
Ausgeben von Daten, die die mindestens eine IEGM-Kurve angeben, die die elektrische Aktivität in dem Gewebe darstellt, die durch die mindestens eine der Katheterelektroden erfasst wird; und
einem zweiten Softwareprogramm, das konfiguriert ist zum:
Empfangen der durch das erste Softwareprogramm ausgegebenen Daten; und
Rendern des mindestens einen IEGM auf die Anzeige über den mindestens einen Teil der anatomischen Karte.

7. System nach Anspruch 1, wobei die Verarbeitungsschaltung konfiguriert ist, um die mindestens eine IEGM-Kurve (i) als eine fortschreitende IEGM-Kurve, die die aktuelle elektrische Aktivität in dem Gewebe darstellt, (ii) als eine statische IEGM-Kurve, die die aufgezeichnete elektrische Aktivität in dem Gewebe darstellt, oder (iii) als eine fortschreitende IEGM-Kurve, die die aufgezeichnete elektrische Aktivität in dem Gewebe darstellt zu rendern.

8. System nach Anspruch 1, wobei die Verarbeitungsschaltung konfiguriert ist zum:
Empfangen einer Benutzereingabe, die einen Punkt auf der anatomischen Karte auswählt; und
Rendern der ausgewählten multiplen IEGM-Kurven auf die Anzeige als Reaktion auf die Benutzereingabe.

9. Computerimplementiertes medizinisches Verfahren, umfassend:
Empfangen von mindestens einem Positionssignal, das eine Position eines distalen Endes eines in eine Herzkammer eines lebenden Subjekts eingeführten Katheters angibt;
Berechnen der Position des distalen Endes des Katheters als Reaktion auf das mindestens eine Positionssignal;
Bewerten einer jeweiligen Berührungsqualität von jeder von mindestens einer Teilmenge von Katheterelektroden des Katheters mit Gewebe der Kammer;
Rendern einer dreidimensionalen (3D) anatomischen Karte der Herzkammer und einer 3D-Darstellung des distalen Endes des Katheters auf eine Anzeige;
Auswählen multipler intrakardialer Elektrogramm-Kurven (IEGM-Kurven) (67), die eine elektrische Aktivität in dem Gewebe darstellen, die durch jeweilige der Katheterelektroden erfasst wird, um auf die Anzeige gerendert zu werden, als Reaktion auf die jeweiligen Berührungsqualität jeder der jeweiligen der Katheterelektroden mit dem Herzgewebe an jeweiligen Stellen; und
Rendern der ausgewählten multiplen intrakardialen Elektrogramm-Kurven (IEGM-Kurven) auf die Anzeige,
wobei das Auswählen der multiplen IEGM-Kurven das Auswählen der multiplen IEGM-Kurven umfasst, die die elektrische Aktivität in dem Gewebe darstellen, die durch n jeweilige der Katheterelektroden erfasst wird, als Reaktion auf die jeweilige Berührungsqualität jeder der n jeweiligen der Katheterelektroden mit dem Herzgewebe, die zu den n höchsten Berührungsqualitäten der mindestens einen Teilmenge der Katheterelektroden gehört.

10. Medizinisches Verfahren nach Anspruch 9, umfassend das Rendern der ausgewählten multiplen intrakardialen Elektrogramm-Kurven (IEGM-Kurven) auf die Anzeige über mindestens einen Teil der anatomischen Karte.

## Revendications

1. Système médical (20), comprenant :
un cathéter (40) conçu pour être inséré dans une cavité d'un cœur d'un sujet vivant, et comportant une extrémité distale (33) comprenant des électrodes de cathéter (48) conçues pour venir en contact avec un tissu au niveau des emplacements respectifs à l'intérieur de la cavité du cœur ;
au moins un capteur de position (50A, 50B) configuré pour fournir au moins un signal de position indiquant une position de l'extrémité distale ;
un affichage (27) ; et
un circuit de traitement (41) configuré pour :
calculer la position de l'extrémité distale du cathéter en réponse à l'au moins un signal de position ;
évaluer une qualité respective du contact de chacune d'au moins un sous-ensemble d'électrodes de cathéter avec le tissu ;
restituer à l'affichage une carte anatomique tridimensionnelle (3D) de la cavité du cœur et une représentation 3D de l'extrémité distale du cathéter ;
sélectionner les multiples tracés d'électrogrammes endocavitaires (IEGM) (67), représentant l'activité électrique dans le tissu qui est détectée par chacune des électrodes respectives de cathéter, à restituer à l'affichage, en réponse à la qualité respective du contact de chacune des électrodes respectives de cathéter avec le tissu cardiaque aux emplacements respectifs ; et
restituer à l'affichage les multiples tracés d'électrogrammes endocavitaires (IEGM) (67) sélectionnés,
dans lequel le circuit de traitement est configuré pour sélectionner les multiples tracés d'IEGM représentant l'activité électrique dans le tissu qui est détectée par un nombre n d'électrodes respectives de cathéter en réponse à la qualité respective du contact de chacune des n électrodes respectives de cathéter avec le tissu du cœur parmi les n qualités de contact les plus élevées de l'au moins un sous-ensemble d'électrodes de cathéter.

2. Système selon la revendication 1, dans lequel le système de circuit de traitement est configuré pour recevoir une entrée utilisateur sélectionnant le sous-ensemble d'électrodes de cathéter.

3. Système selon la revendication 1, dans lequel le circuit de traitement est configuré pour :
recevoir une entrée utilisateur sélectionnant une région de la carte anatomique ; et
restituer à l'affichage les multiples tracés d'IEGM sélectionnées représentant l'activité électrique dans le tissu qui est détectée par des électrodes respectives de cathéter en réponse : aux électrodes respectives de cathéter étant situées à proximité de la région sélectionnée de la carte anatomique ; et à la qualité respective du contact des électrodes respectives de cathéter avec le tissu cardiaque au niveau des emplacements respectifs.

4. Système médical selon la revendication 1, dans lequel le circuit de traitement est configuré pour restituer les multiples tracés d'électrogrammes endocavitaires (IEGM) (67) sélectionnés à l'affichage sur au moins une partie de la carte anatomique.

5. Système selon la revendication 1, dans lequel le circuit de traitement est configuré pour déplacer une position de l'au moins un tracé d'IEGM restitué sur l'affichage afin de suivre le mouvement de la position de l'extrémité distale du cathéter.

6. Système selon la revendication 1, dans lequel le circuit de traitement est configuré pour exécuter :
un premier programme logiciel configuré pour :
calculer la position de l'extrémité distale du cathéter en réponse à l'au moins un signal de position ; et
restituer à l'affichage la carte anatomique de la cavité du cœur et la représentation de l'extrémité distale du cathéter ; et
produire des données indiquant l'au moins un tracé d'IEGM représentant l'activité électrique dans le tissu qui est détectée par l'au moins une des électrodes de cathéter ; et
un second programme logiciel configuré pour :
recevoir les données produites par le premier programme logiciel ; et
restituer à l'affichage, sur l'au moins une partie de la carte anatomique, l'au moins un IEGM.

7. Système selon la revendication 1, dans lequel le circuit de traitement est configuré pour restituer l'au moins un tracé d'IEGM (i) comme un tracé d'IEGM progressif représentant l'activité électrique actuelle dans le tissu, (ii) comme un tracé d'IEGM statique représentant l'activité électrique enregistrée dans le tissu, ou (iii) comme un tracé d'IEGM progressif représentant l'activité électrique enregistrée dans le tissu.

8. Système selon la revendication 1, dans lequel le circuit de traitement est configuré pour :
recevoir une entrée utilisateur sélectionnant un point sur la carte anatomique ; et
restituer à l'affichage les multiples tracés d'IEGM sélectionnés en réponse à l'entrée utilisateur.

9. Procédé médical mis en œuvre par ordinateur, comprenant :
la réception d'au moins un signal de position indiquant une position d'une extrémité distale d'un cathéter inséré dans une cavité d'un cœur d'un sujet vivant ;
le calcul de la position de l'extrémité distale du cathéter en réponse à l'au moins un signal de position ;
l'évaluation d'une qualité respective du contact de chacune d'au moins un sous-ensemble d'électrodes de cathéter avec le tissu de la cavité ;
la restitution à un affichage d'une carte anatomique tridimensionnelle (3D) de la cavité du cœur et d'une représentation 3D de l'extrémité distale du cathéter ;
la sélection des multiples tracés d'électrogrammes endocavitaires (IEGM) (67), représentant l'activité électrique dans le tissu qui est détectée par chacune des électrodes respectives de cathéter, à restituer à l'affichage, en réponse à la qualité respective du contact de chacune des électrodes respectives de cathéter avec le tissu cardiaque à des emplacements respectifs ; et
la restitution à l'affichage des multiples tracés d'électrogrammes endocavitaires (IEGM) sélectionnées,
dans lequel la sélection des multiples tracés **d'IEGM** comprend la sélection des multiples tracés **d'IEGM** représentant l'activité électrique dans le tissu qui est détectée par un nombre n d'électrodes respectives de cathéter en réponse à la qualité respective du contact de chacune des n électrodes respectives de cathéter avec le tissu du cœur parmi les n qualités de contact les plus élevées de l'au moins un sous-ensemble d'électrodes de cathéter.

10. Procédé médical selon la revendication 9, comprenant la restitution des multiples tracés d'électrogrammes endocavitaires (IEGM) sélectionnées à l'affichage sur au moins une partie de la carte anatomique.
